# EUROPEAN PATENT SPECIFICATION

(11) **EP 2 923 293 B1**
(45) Date of publication and mention of the grant of the patent: **26.05.2021**
(21) Application number: 13711221.5
(22) Date of filing: 07.03.2013
(51) Int. Cl.: G16B 30/10, G16B 50/30

(54) **EFFICIENT COMPARISON OF POLYNUCLEOTIDE SEQUENCES**
EFFIZIENTER VERGLEICH VON POLYNUKLEOTIDSEQUENZEN
COMPARAISON EFFICACE DE SÉQUENCES POLYNUCLÉOTIDIQUES

(30) Priority: 26.11.2012 US 201261729791 P
(43) Date of publication of application: 30.09.2015
(73) Proprietor: Illumina, Inc., San Diego, CA 92122 (US)
(72) Inventor: MANN, Tobias, MI 48103 (US)
(74) Representative: Mewburn Ellis LLP
(86) International application number: PCT/US2013/029653
(87) International publication number: WO 2014/081456

(56) References cited:
- YOICHI TAKENAKA ET AL: "Perfect Hamming code with a hash table for faster genome mapping", BMC GENOMICS, BIOMED CENTRAL LTD, LONDON, UK, vol. 12, no. Suppl 3, 30 November 2011 (2011-11-30), page S8, XP021111629, ISSN: 1471-2164, DOI: 10.1186/1471-2164-12-S3-S8 cited in the application
- H. LI ET AL: "A survey of sequence alignment algorithms for next-generation sequencing", BRIEFINGS IN BIOINFORMATICS, vol. 11, no. 5, 11 May 2010 (2010-05-11), pages 473-483, XP055085554, ISSN: 1467-5463, DOI: 10.1093/bib/bbq015
- STEVE HOFFMANN ET AL: "Fast Mapping of Short Sequences with Mismatches, Insertions and Deletions Using Index Structures", PLOS COMPUTATIONAL BIOLOGY, vol. 5, no. 9, 11 September 2009 (2009-09-11), page e1000502, XP055070487, DOI: 10.1371/journal.pcbi.1000502

### SEQUENCE LISTING

<110> ILLUMINA, INC. Mann, Tobias
<120> EFFICIENT COMPARISON OF POLYNUCLEOTIDE SEQUENCES
<130> ILLINC.208WO
<150> 61/729,791
   <151> 2012-11-26
<160> 8
<170> FastSEQ for Windows Version 4.0
<210> 1
   <211> 20
   <212> DNA
   <213> Artificial Sequence
<220>
   <223> Artificially designed polynucleotide
<400> 1
   ccatgatatt tcaatatatt 20
<210> 2
   <211> 15
   <212> DNA
   <213> Artificial Sequence
<220>
   <223> Artificially designed polynucleotide
<400> 2
   gtaagcgaac tgata 15
<210> 3
   <211> 15
   <212> DNA
   <213> Artificial Sequence
<220>
   <223> Artificially designed polynucleotide
<400> 3
   gtaaccgacc tgatc 15
<210> 4
   <211> 15
   <212> DNA
   <213> Artificial Sequence
<220>
   <223> Artificially designed polynucleotide
<400> 4
   ataagtgaac tgaga 15
<210> 5
   <211> 15
   <212> DNA
   <213> Artificial Sequence
<220>
   <223> Artificially designed polynucleotide
<400> 5
   ggaagcgagc agata 15
<210> 6
   <211> 20
   <212> DNA
   <213> Artificial Sequence
<220>
   <223> Artificially designed polynucleotide
<400> 6
   cttgctttgg cttgctgctt 20
<210> 7
   <211> 20
   <212> DNA
   <213> Artificial Sequence
<220>
   <223> Artificially designed polynucleotide
<400> 7
   ttggggattg actgggttcc 20
<210> 8
   <211> 20
   <212> DNA
   <213> Artificial Sequence
<220>
   <223> Artificially designed polynucleotide
<400> 8
   cagcccaccc ctctcacctg 20

## Description

### FIELD OF THE INVENTION

The disclosure relates to rapid identification of oligonucleotides in nucleotide sequence datasets.

### BACKGROUND OF THE INVENTION

As high throughput nucleotide sequencing becomes a more routine tool in science and medicine, there is a need for rapid sequence analysis tools. In particular, there is a need for methods and devices that allow one to rapidly search for a large number of unique or fairly unique oligonucleotide sequences in a genomic data set. Current sequence analysis tools can take as long as 40 minutes or longer to identify a given set of relatively short polynucleotide sequences in a database of stored genomic sequences.

Some current approaches involve identifying markers within the genomic sequences in a database, and then creating an index of those markers. The system divides the sequence reads into short oligonucleotides, such as 15mers, 20mers or 25mers for alignment against this index of stored genomic markers. However, for 25 million 20mers that are taken from a sample sequence, it can take up to 40 minutes or more to align them to an indexed 65 million oligonucleotide marker set of stored genomic markers.

Alternately, one can generate an index of polynucleotide sequences that are being determined in a sequencing reaction and then search that index against a stored dataset of 65 million oligonucleotide markers from a genome. This process can take about 20 minutes of formatting and 20 minutes of searching at current computing capacity.

Neither of these options are particularly attractive, as both involve substantial amounts of time. Furthermore, current alignment tools such as "bowtie" or "bwa" are computationally intensive and require pre-built indices and extensive post-processing efforts. Additionally, current techniques relying on prebuilt techniques do not work well with customers that want to align their sequences against custom developed genomes. Building indices for custom genomes using present techniques slows the process even further and occupies substantial computer space.
Tanenaka et al. (BMC Genomics201112 (Suppl 3) :S8, https://doi.org/10.1186/1471-2164-12-S3-S8) describes a hash based method for genome mapping in which DNA subsequences are divided into equivalence classes by using a perfect Hamming code.

### SUMMARY OF THE INVENTION

Accordingly, in one aspect the present invention provides a computer implemented method of aligning a first 20mer oligonucleotide sequence to all 20mer fragments of a nucleotide data set comprising:
indexing all 5mer fragments of oligonucleotides into a first index of perfect Hamming code 5mers, wherein each said perfect Hamming code 5mer has a set of 15 additional 5mers which form an equivalence class with said perfect Hamming code 5mer such that each additional 5mer of said equivalence class differs from said perfect Hamming code 5mer by a Hamming code distance of 1, and wherein each said perfect Hamming code 5mer differs from all other perfect Hamming code 5mers by at least a Hamming code distance of 3;
configuring said first index into a first level of a four level trie having a branching factor of 64 such that each leaf of said first level represents a different perfect Hamming code 5mer of said first index and the equivalence class said perfect Hamming 5mer defines;
configuring said trie such that each leaf of said first level is a trunk of a second index on a second level of said trie, said second index having the same structure as said first index, each leaf of said second level is a trunk of a third index on a third level of said trie, said third index having the same structure as said first index, and each leaf of said third level is a trunk of a fourth index on a fourth level of said trie, said fourth index having the same structure as said first index, such that said trie has a depth of 4 levels and 64⁴ leaves at said fourth level, and such that each 20mer fragment of said nucleotide data set maps to only one path from a 4th level leaf to the first level trunk of said trie, and wherein each unique trie path corresponds to a concatenation of four consecutive perfect Hamming code 5mers to form a 20mer fragment of said trie path;
assigning each 20mer fragment of said nucleotide data set to a unique trie path; identifying a trie path corresponding to said first 20mer oligonucleotide sequence; and
comparing said 20mer fragments of said trie path to said first 20mer oligonucleotide sequence, wherein said comparing comprises:
comparing positions 1-5 of said first 20mer oligonucleotide sequence to the thirty equivalence classes at said first level each having at least one member which differs from at least one member of the equivalence class with positions 1-5 of said first 20mer oligonucleotide sequence by a Hamming distance of 1,
comparing positions 6-10 of said first 20mer oligonucleotide sequence to the thirty equivalence classes at said second level each having at least one member which differs from at least one member of the equivalence class with positions 6-10 of said first 20mer oligonucleotide sequence by a Hamming distance of 1,
comparing positions 11-15 of said first 20mer oligonucleotide sequence to the thirty equivalence classes at said third level each having at least one member which differs from at least one member of the equivalence class with positions 11-15 of said first 20mer oligonucleotide sequence by a Hamming distance of 1, and
comparing positions 16-20 of said first 20mer oligonucleotide sequence to the thirty equivalence classes at said fourth level each having at least one member which differs from at least one member of the equivalence class with positions 16-20 of said first 20mer oligonucleotide sequence by a Hamming distance of 1.

In some aspects the first 20mer oligonucleotide maps to a unique region of a genomic reference sequence and the oligonucleotide spans a single nucleotide polymorphic site.

In some aspects the method further comprises discarding the sample if the first 20mer oligonucleotide sequence is not identically present in the sample sequence. In some aspects the first 20mer oligonucleotide sequence is derived from a sample, and the nucleic acid data set is labeled to be derived from the sample.

In some aspects the nucleic acid data set comprises sequence from an incompletely sequenced nucleotide sample.

In some aspects the nucleotide sample is being sequenced concurrently with execution of the method.

In some aspects the method determines whether the nucleic acid data set and the 20mer oligonucleotide sequence are derived from different sources.

In some aspects the first 20mer oligonucleotide sequence is determined by hybridization of a nucleic acid sample to a nucleic acid probe complementary to the first 20mer oligonucleotide at the single nucleotide polymorphic site.

In some aspects the first 20mer oligonucleotide sequence is determined using microarray hybridization. In some aspects the oligonucleotide sequence is determined using a sequencing method.

In some aspects a region comprising the oligonucleotide single nucleotide polymorphic site is amplified in a polymerase chain reaction.

In some aspects the first 20mer oligonucleotide maps to a region that undergoes copy number variation in a population, and hits to sequence identical to the first 20mer oligonucleotide are indicative of a copy number of the homologous region in the sample.

The method may further comprise searching the genomic sequence using a second 20mer oligonucleotide sequence that is homologous to a region having a copy number that does not co-vary with the copy number variant region.

The 20mer oligonucleotide search results may be indicative of the copy number of the homologous regions.

The method may comprise calculating a ratio of the copy number variation at a region.

Alignment results may be used to determine the identity of duplicate nucleotide samples.

The multiple duplicate samples may be mislabeled.

In a further aspect, the present invention provides a computer based system for aligning a first 20mer oligonucleotide sequence to all 20mer fragments of a nucleotide dataset as set out in claim 9.

### BRIEF DESCRIPTION OF THE DRAWINGS

Figure 1 is a block diagram of an embodiment of a polynucleotide comparison system.

Figure 2 is a flow diagram of one embodiment of a process for comparing nucleotide sequences.

Figure 3 is a block diagram of an equivalence class according to one embodiment. The sequence GCGTT at the center is a Perfect Hamming Code (PHC) of the illustrated equivalence class.

Figure 4 is a diagram of a four level trie of indexed PHC sequences made of five nucleotides (5mers). Only 3 of the 64 leaves at each level are shown, and only a single leaf at each level is shown to form the trunk of the next level. The figure depicts the path representing the 20mer CCATGATATTTCAATATATT (SEQ ID NO:1). The actual trie would have over 16 million leaves.

Figure 5 is a graph showing the results of a copy number determination through an embodiment of the method.

Figure 6 is a block diagram showing a simplified depiction of three 20mer leaves of a one-level trie (the other 61 leaves are not shown). The sequences listed are as follows: CTTGCTTTGGCTTGCTGCTT (SEQ ID NO:6); TTGGGGATTGACTGGGTTCC (SEQ ID NO:7); CAGCCCACCCCTCTCACCTG (SEQ ID NO:8). Also shown in Figure 6 is an all by all comparison of a needle index with a haystack index.

Figure 7 is a simplified block diagram depicting two equivalence classes having a Hamming code distance of 3 to one another.

Figure 8 shows one embodiment of a process for filling input buffers and processing results in a sequence comparison system.

Figure 9 is a block diagram showing an embodiment of a process for determining results of a search for polynucleotide "needles" within a target "haystack" database of sequences.

### DETAILED DESCRIPTION

The present disclosure relates to methods and systems configured to rapidly search for oligonucleotide sequences in a dataset of polynucleotides, such as a genomic sequence dataset. Also described is a system for rapidly determining if a DNA sample being sequenced is the correct sample. In this system, a biological sample of DNA is provided. That DNA is processed according to known methods and run over a microarray to identify unique single nucleotide polymorphisms (SNPs) within the biological sample. That data, from 100, 1000, 10,000, 100,000, 1,000,000, or more SNP sites in the biological sample is stored in the analysis system, or laboratory information management system (LIMS). Thus, SNP data for that particular biological sample is now known. In this system, the same biological sample is then run on a sequencer to determine the exact DNA sequence of some or all of that user's DNA. However, during the sequencing reaction, it is possible that incorrect samples are applied to the sequencer, or other errors are made so that the sequencer is not properly sequencing the desired sample. Thus, this system provides a way of checking, in real time, whether the proper sample was placed on the sequencer.

As the sequence data is being generated by the sequencer, there are hundreds, thousands, tens of thousands, or more sequencing reactions occurring at the same time in the sequencer. In embodiments of the invention, each of these reactions is monitored, and oligonucleotide sequences from the sequencer are compared to the sequences found in the earlier microarray data to ensure that there is concordance. Thus, the same person would have the same unique SNPs found in their DNA from the microarray experiment as would be found in the sequence data. Should the system find that a particular unique SNP generated from the sequencer did not match with the same SNP determined from the microarray, the system could set an error flag and notify the operator that a sample mistake may have been made. This can prevent the wrong sample from being sequenced, and save on valuable reagents that would otherwise have been used to sequence the incorrect sample.

A challenge addressed by embodiments of the present disclosure is that of comparing millions of unique sequences against a panel of millions of unique SNP regions in a fast, and even real time manner in order to detect errors. In some embodiments, the described system can align a dataset of up to 65 million unique polynucleotides against a set of 25 million 20mer oligonucleotides in less than 20 minutes. The polynucleotides in the dataset and the oligonucleotides are both 20 nucleotides in length ("20mers"). Smaller-scale applications, such as matching 20-30 20mer oligonucleotide probes against a genomic sequence dataset of 65 million polynucleotides can be accomplished in less than 1 minute.

In some embodiments, disclosed herein are methods and systems for the rapid identification of oligonucleotide 'needles' in large polynucleotide datasets, termed herein 'haystacks'. In some embodiments this rapid identification is effected through a process of rapid, efficient indexing followed by a greatly facilitated comparison of the needle sequences to the indexed haystack sequences.

### Perfect Hamming Code 5mers

The invention uses the mathematical concept of perfect Hamming codes to index a set of oligonucleotides into an index that has the properties of a perfect Hamming code ('PHC'). A set of 64 oligonucleotide sequences that are five nucleotides in length (5mers) can be generated wherein every 5mer oligonucleotide sequence not in the 64 5mer set differs from one and only one of the other 64 5mers in the perfect Hamming code set by one and only one base substittion, and has more base substitutions when compared to all other members of the 5-mer set. See Takenaka, et al, (2011) "Perfect Hamming code with a hash table for faster genomic mapping" BMC Genomics 12(S3):S8. This property of forming a perfect Hamming code is unique to oligonucleotides of length k, wherein (3^{k}+1)/4 is a whole number. Nontrivial solutions for this equation include 5 and 21.

The oligonucleotides which differ from one of the 64 Hamming code solution 5mers by a single base change (that is, having a 'Hamming distance' of 1) may be described as falling into the same equivalence class as that of the Hamming code solution 5mer. See Example 1, depicting equivalence classes for three perfect hamming code (PHC) 5mer sequences, which also include the PHC 5mer itself.

A result of this structure yields an approach to search for approximate matches to 5mer sequences on the basis of the equivalence classes generated by the perfect hamming code. If the system must find 5-mers with at most one mismatch to a query 5mer, it can do this on the basis of the equivalence classes by examining equivalence classes whose perfect hamming code template has a hamming distance of 3 to the perfect hamming code template for the original query 5mer. One property of the perfect hamming code is that each member will have hamming distance 3 to exactly 30 of the 64 members of the perfect hamming code. Restated, a non PHC 5mer in a given equivalence class (having a Hamming distance separated from the PHC 5mer of its equivalence class by 1) can only match sequences with at most one error if those sequences are in one of 30 equivalence classes defined by the PHC template for the original query sequence.

### Constructing indices of concatenated 5mers

It may be advantageous to align sequences that are longer than five nucleotides when searching for unique genomic sequences. Thus, systems described herein have been developed that concatenate 5mer PHC oligonucleotides into longer sequences of 10mers, 15mers, 20mers, 25mers or larger sequences in intervals of 5 nucleotides. According to the invention, a concatenated 20mer oligonucleotide made up of four sets of Hamming code solution 5mers, for which each individual 5mer falls into an equivalence class of the corresponding Hamming code 5mer used to form the concatenated 20mer, may be described as falling into the same equivalence class as the concatenated 20mer of four Hamming code solution 5mers. A 20mer made from four PHC 5mers may differ by as many as four bases from the members of its equivalence class, so long as there is only one difference in the first 5 base interval, 1 in the second 5 base interval, one in the third 5 base interval, and 1 in the fourth 5 base interval, each interval corresponding to a PHC 5mer from which the concatenated 20mer was built.

The 64 sets of equivalence classes each corresponding to a PHC 5mer can be depicted as endpoints, or 'leaves', branching from a single trunk of a decision 'trie'. Thus any 5mer at the base of the trie can be placed into the bin represented by a leaf at the end of each branch based on having a sequence within the equivalence class of that leaf.

Each endpoint leaf of this first branch level in turn serves as a trunk for a new set of 64 branches. Thus the second level of the 'trie' has 64x64, or 4096 leaves as endpoints. Thus any 10mer can rapidly be mapped to a single level two leaf, based on the placement of its first 5mer into an equivalence class of a leaf at the first level, and then the placement of its second 5mer into a leaf that branches from the leaf/trunk of the first level equivalence class.

Notably, although there are 4096 equivalence class leaves for this two-level tree representing all possible 10mers, only 128 leaves need to be evaluated - 64 form the first level and then the sixty four of the second level that branch from the equivalence class leaf identified from the first level. Thus the configuration of the sequence space of all possible 10mer sequences into equivalence classes based on concatenated PHC 5mer leaves dramatically reduces the amount of sequence space that one needs to search to place a given 10mer into an equivalence class consisting of 10mers which differ from it by as many as 2 bases.

Each endpoint leaf of the second branch level in turn serves as a trunk for a new set of 64 branches. Thus the third level of the 'trie' has 64x64x64, or 262144 leaves as endpoints.

Any 15mer can rapidly be mapped to a single level three leaf, based on the placement of its first 5mer into an equivalence class of a leaf at the first level, placement of its second 5mer into a leaf that branches from the leaf/trunk of the first level equivalence class, and placement of its third 5mer into a leaf that branches from the leaf/trunk of the second level equivalence class.

Similarly, each endpoint leaf of the third branch level in turn serves as a trunk for a new set of 64 branches. Thus the fourth level of the 'trie' has 64x64x64x64, or 16,777,216 leaves as endpoints.

However, any 20mer can rapidly be mapped to a single level four leaf, based on the placement of its first 5mer into an equivalence class of a leaf at the first level, placement of its second 5mer into a leaf that branches from the leaf/trunk of the first level equivalence class, placement of its third 5mer into a leaf that branches from the leaf/trunk of the second level equivalence class, and placement of its fourth 5mer into a leaf that branches from the leaf/trunk of the third level equivalence class.

Thus, placement of a 20mer into one of over 16 million equivalence classes may be accomplished by evaluating a mere 256 leaf/5mer equivalence classes - 64 at leach level.

### Comparing leaves of multiple trie indices

In the present invention, nucleotide data sets are divided into concatenated 5mers and configured into multilevel trie indices. The concatenated oligonucleotide length is a 20mer.

In some embodiments a configured nucleotide data set 'haystack' is to be compared to a set of oligonucleotide probe or 'needle' sequences. In some embodiments both the haystack and the needle sequence data sets are configured into 'trie's of equivalence classes, such that the fourth level 'leaves' may be rapidly compared to one another.

In some embodiments 20mers differing from a needle 20mer by at most 1 single nucleotide may be detected, such as 20mers which span a single SNP which may be indicative of the identity of a nucleotide sample. This comparison involves, for a single 20mer needle sequence, analysis of a number of hay leaf/equivalence classes equal to the 1) equivalence class of the 20mer needle itself, 2) the 30 equivalence classes having a 5mer in their first position that differs by one from a non PHC 5mer of the needle first position 5mer's equivalence class, 3) the 30 equivalence classes having a 5mer in their second position that differs by one from a non PHC 5mer of the needle second position 5mer's equivalence class, 4) the 30 equivalence classes having a 5mer in their third position that differs by one from a non PHC 5mer of the needle third position 5mer's equivalence class, and 5) the 30 equivalence classes having a 5mer in their fourth position that differs by one from a non PHC 5mer of the needle fourth position 5mer's equivalence class.

Thus, for example, an indexed 20mer needle may be matched to a sequence in a leaf of an indexed haystack trie of 20mer sequences by examining a mere 121 sequences out of the over 16 million haystack trie leaf bins.

### Identifying useful needle sets

In some embodiments as disclosed herein, a set of 65 million 20mers is evaluated against a reference data set such as the human genome to identify 20mers that are unique or fairly unique to a genomic data set and that span variable regions such as single nucleotide polymorphisms. Such 20mer oligos have as a characteristic the trait that they differ from a homologous sequence in another individual's genome by a Hamming distance of 1.

This reduced set of 20mers has the novel, useful trait that their homologous sequence in an unknown sample can be identified in an indexed human genome dataset by evaluating the contents of only 121 leaf/equivalence class bins out of the over 16 million theoretically extant bins in such a dataset.

### Implementations of the methods, indices and computer-based search algorithms disclosed herein

Given this refined set of 20mer oligonucleotide sequences, one can rapidly assay nucleotide sequence data, such as genomic sequence as it is being generated in a sequencing run, to determine whether or not the sequence data is properly labeled and identified. Such a technique is a valuable tool for the identification of, for example, mislabeled genomic samples early in the sequencing process to prevent the loss of further reagents, computing capacity and time. The technique can be valuable in other contexts as well including, but not limited to, forensics (e.g. identification of individuals based on nucleic acid sequence similarities to one or more forensic reference sequence), diagnostics (e.g. determining risk of a clinical condition or disease based on nucleic acid sequence similarities to one or more diagnostic reference sequences) or prognostics (e.g. predicting a clinical outcome based on nucleic acid sequence similarities to one or more prognostic reference sequences), or metagenomics (e.g. identifying the species composition of microbial mixtures), or DNA barcoding (e.g. identifying organisms based on unique tag sequences)

In some embodiments, sequence comprising a useful set of 20mer selected so as to map to a unique region of the human reference genome and to span a single SNP are selected. In some embodiments a microarray comprising at least one of these 20mer sequences, or a subset thereof sufficient to distinguish the identity a nucleotide at said SNP in a sample, is contacted to a sample under conditions known in the art to facilitate determination of the SNP nucleotide at said position in said 20mer sequence of said sample. In some embodiments the identity of a single SNP in a genomic sequence spanning a 20mer of interest is determined using methods other than microarray analysis, such as sequencing of a polymerase chain reaction generated amplicons spanning the SNP, or differential hybridization of a probe to a polymerase chain reaction generated amplicons spanning the SNP. Other methods of determining the identity of a nucleotide at an SNP position are consistent with the methods disclosed herein.

In some embodiments a 20mer corresponding to the sequence of the useful 20mer with the SNP position specified is used as a needle to assay for the presence of said sequence, or to identify single nucleotide variants of said sequence, in a genomic sample. In some embodiments, the sequence to be assayed is believed to be derived from the same sample that was applied to a microarray, such that the identification of the specified 20mer sequence may be used as a means of confirming that the sample being sequenced is correctly labeled (or otherwise correctly correlated or cross-referenced with the sample used for microarray analysis).

In some embodiments, the 20mer searching process is fast enough so that a verification of the identity of a sequence may be made while the sequencing of the sample is in progress. In some embodiments a sequencing reaction such as a wholegenome sequencing reaction being performed on a sample may be terminated if said sample is determined by the methods disclosed herein not to match its expected sequence. In some embodiments, genomic sequence determined by the methods disclosed herein not to match its expected sequence is withdrawn from further analysis, for example under suspicion that said genomic sample has been mislabeled. Alternatively, the sequencing reaction can be paused, for example, while sample tracking is sorted out, and subsequently restarted. For diagnostic, prognostic or forensic applications, sequencing can be terminated or paused once similarity between test and reference nucleic acid sequences has been determined at a desired or predetermined confidence level.

In some embodiments the method disclosed herein may be used to evaluate copy number variation in a genomic sample. Copy number variation is presently determined using, for example, differential hybridization intensities on microarrays. In some embodiments of the present invention, 20mer oligonucleotide sequences homologous to genomic regions where relative copy number determinations are desired may be selected. Genomic sequence for which copy number determinations are to be made may be indexed and searched using the 20mer needles as discussed. In some embodiments, the number of hits to a given 20mer needle in a genomic sample sequence is indicative of the relative copy number of that genomic region. Accordingly, methods set forth herein can be used to identify an aneuploidy, for example, in a fetal nucleic acid sample obtained from a pregnant female, or in a nucleic acid sample obtained from an individual suspected of having cancer.

Using the methods of the present disclosure, copy number is evaluated using quantitative digital methods and discrete statistics, rather than quantification of analog signal processing data.

In some embodiments the copy number determined corresponds to the ploidy of a given genomic region or locus. In some embodiments the region is of substantial significance to the health of an individual with which a sample is associated. For example, aneuploidy detected for a fetal nucleic acid sample can be indicative of mental retardation or other developmental delays. Aneuploidy can also be indicative of cancer.

In some embodiments, the methods disclosed herein may be used to correctly identify a number of samples which are being run simultaneously and for which the identities of the samples have been confused, for example by adding a sample to the wrong flow cell in a multiplex sequencer. In some embodiments the samples are run in duplicate. In some embodiments 20mer oligonucleotides may be used to identify homozygous mismatches with the human genome reference sequence in a given sample that are distinctive to a given pair of samples, such as homozygous disagreements to a reference, such that all samples can be accurately paired.

### Operational parameters of the methods and computer-based search algorithms disclosed herein

In some embodiments, the computational methods disclosed herein may be performed on a graphics processing unit. In some embodiments the computational methods disclosed herein operate from bcl files. In some embodiments operation of the computer-based methods disclosed herein operate on bcl files and require no intermediate file generation. In some embodiments there may be separate implementations for full and sparse indices, or for GPU and multi-core searchers. In some embodiments uncompressed, gzipped or bzipped FASTA and FASTQ files may be read. In some embodiments, algorithms are designed to parallelize well for short reads or long chromosomal sequences. Some embodiments comprise seqanized design style and/or highly parallelized IO/searching.

In some embodiments, the computational burden of performing the methods and computer based algorithms disclosed herein scales as O (N) rather than O (N logN) in methods available in the art. Consequently, the methods and computer based algorithms disclosed herein may be performed more rapidly with substantially less computer computational capacity and memory than methods and computer based algorithms known in the art. This advantage is particularly true for large values of N, wherein N is the number of 20-mers to be analyzed - the sorting efficiency is of greatest benefit when there are a lot of needles or a lot of haystacks to analyze.

### Operational Low Level Architecture of the Methods disclosed herein

In some embodiments, sequences are split into 20mer oligonucleotide needle sequences and then binary encoded, for example such that the bases a, c, g, and t correspond to 00, 01, 10, and 11, respectively. 20mers may be encoded as 64 bit unsigned integers, and each short unsigned integer may contain a 5mer, such that each encoded unsigned short integer is also a number from 0 to 1024.

For an arbitrary 20mer, a node index may be computed as follows. Sort the 64 PHC template 5mers and the associated 5mers of each equivalence class. Place the first 5mer of the arbitrary 20mer into an equivalence class by evaluating the 1024 5mers in that set. Then repeat the process for the second 5mer, third 3mer and fourth 5mer, placing each into a 5mer equivalence class so that the arbitrary 20mer can be placed into four concatenated equivalence classes.

To perform a Hamming distance 1 search on a given node index (that is, to identify all oligonucleotides which differ from a given oligonucleotide by 1), one may proceed as follows. Reconstruct the concatenated 5mers constituents of said node index. Then compute a lookup table for each 5mer wherein said table comprises all of the 30 Hamming distance 1 search neighbors of said 5mer sequence. The data may be processed by replacing bits and computing the resulting leaf offset, and adding the computed offset to a list. The process may be repeated for each 5mer in a concatenated oligonucleotide such as a 20mer.

Building this neighbor list requires a small number of integer additions and table lookups. For a 20mer, a neighborhood of 121 equivalence classes must be evaluated to determine the set of Hamming distance 1 oligonucleotides. Keeping in mind that there are over 16 million 20mer equivalence classes, reduction of the number to be searched to 121 represents a major improvement over current methods.

A workflow arising out of the methods and computational algorithms disclosed herein is as follows. For a given needle index and haystack index generated using the methods and computer based algorithms disclosed herein, a workflow comprises filling an input buffer and initializing a results buffer, processing the buffers on CPUs, and reading and processing the results buffer. Alternately, one may copy buffers to a GPU, process the buffers on a GPU, copy the buffers from a GPU, and read and process the results buffer.

The search buffers for a neighborhood search may be set up by using a needle index to copy the needle 20mers into a local buffer and using a haystack index to copy the haystack 20mers into a local buffer. A local results buffer may be initialized wherein each needle index is compared to all calculated haystack indices. The sequences of each indexed 20mer needle/haystack pair may be compared directly or their integer values calculated above may be compared using the 'xor' operation. A difference of 0 indicates that the sequences are identical. A difference of 1 in integer value indicates a Hamming distance of 1, thus 1 base difference between the oligos. A difference of 2 may indicate a Hamming distance of 1 or 2, and may be further investigated to determine the actual Hamming distance. A difference of 3 or greater indicates a Hamming code distance of greater than or equal to 2.

In cases where the difference is 2, a Hamming distance of 1 may be found if the nucleotide substitution involves substitutions with two-bit changes as discussed above. The possible changes are A/T (00 to 11) and G/C (10 to 01). These situations can be distinguished from other situations resulting in a difference of 2 by ensuring that the least significant nonzero bit in the xor result is at an even position, and the adjacent higher bit is set.

For example, if analysis indicates that the lowest nonzero bit is odd, then the difference cannot result from a single substitution. Similarly, if the next bit is not set, then we cannot be looking at a single substitution.

Further understanding of embodiments of the disclosure may be had by a more detailed examination of the accompanying figures.

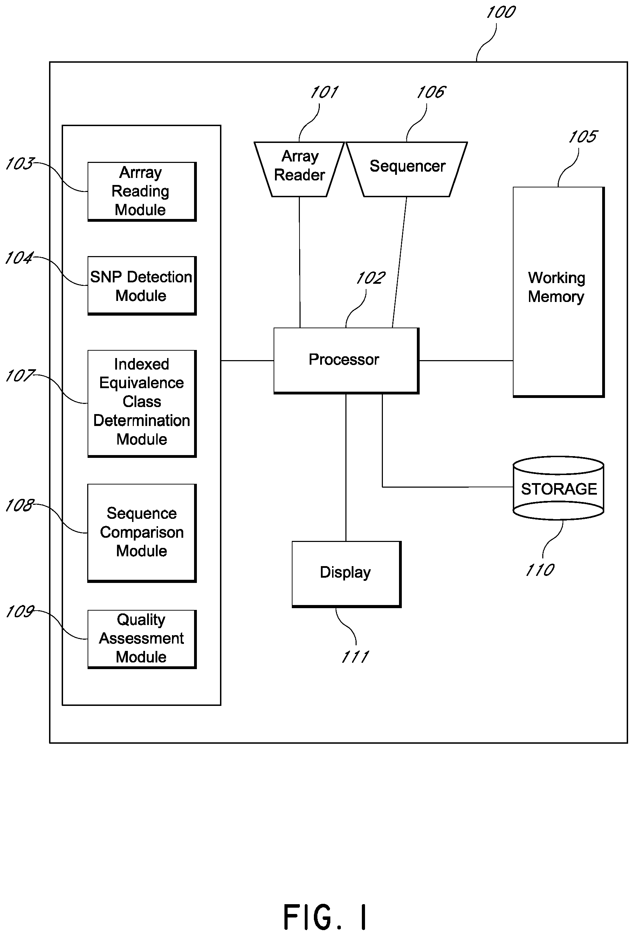

Figure 1 depicts a system 100 configured to carry out some embodiments of the present disclosure. The system 100 includes a connection to an array reader 101 that can read microarray hybridization results. Microarrays and array readers known in the art may be used, for example the Illumina iScan, HiScan® or BEADEXPRESS® system. The microarrays to be read may comprise oligonucleotide sequences that selectively bind to specific alleles at single nucleotide polymorphic (SNP) sites. Thus in some embodiments the array reader will receive signals indicative of the identity of a base at an SNP position in a given oligonucleotide being assayed, such as a 20mer spanning an SNP in an otherwise unique region of a reference genome. In one embodiment, the sequence is a human genome reference sequence, meaning that the sequence is unique in the genome of human beings and includes at a polymorphic region that differs by a single base pair between different alleles. In some embodiments the oligonucleotide sequence on the microarray is configured to selectively bind to a particular SNP base but is not the same length as the selected 20mer to be used in downstream processes, as discussed below.

Data from the Array Reader 101 is passed to a Processor 102 which evaluates and processes any data arriving from the array. Stored within a memory of the system 100, and configured to be ready the processor 102, is an Array Reading Module 103 which converts the signal intensities reported by the Array Reader 101 into digitally accessible data. This data may be presented to a SNP Detection Module 104 which identifies the base at the SNP position or positions of interest. The SNP position data, as well as 20mer full length data, may be stored on a Working Memory module 105 connected to the Processor 102.

A Sequencer 106 may also be in communication with the Processor 102. The sequencer may generate nucleic acid sequence data, such as genomic sequence data, from a biological sample of DNA taken from a human being. In some embodiments the sample from which Array data is taken is the same sample that is being sequenced, such that the data being sequenced can be confirmed by the data gathered from the microarray. The data generated by the sequencer may be provided to the processor for immediate analysis and may be concurrently or later stored on the Working Memory 105.

An Indexed Equivalence Class Determination Module 107 may also be present in the system 100, and configured to run on the Processor 102. The Indexed Equivalence Class Determination Module 107 may include instructions for identifying 64 member PHC 5mer sets of oligonucleotides. The module 107 can also identify, for each PHC member, the remaining 15 5mers that have a perfect Hamming code distance of 1 from each PHC 5mer. The Indexed Equivalence Class Determination Module may also serve to concatenate PHC 5mers to generate longer oligonucleotides. In some embodiments, the Indexed Equivalence Class Determination Module is configured to concatenate four of the PHC 5mers together to generate a 20mers oligonucleotide that is used for further analysis. The Indexed Equivalence Class oligonucleotides may be analyzed directly or may be stored in the Working Memory 105.

A Sequence Comparison Module 108 may also be included within the system 100, and configured to run on the Processor 102. The Sequence Comparison Module 108 may perform a sequence comparison, either directly or through the analysis of digital depictions of the oligonucleotide data, such as the sequence comparison approaches disclosed herein.

A Quality Assessment Module 109 may also be included within the system 100 and configured to run on the Processor 102. The Quality Assessment Module 109 may include instructions to evaluate the results generated by the Sequence Comparison Module 108 to generate a report indicating whether a particular 20mer oligonucleotide sequence determined by contacting a sample to an Array Reader 101 is consistent with the results obtained by contacting a sample to a Sequencer 106, as mentioned above. The Quality Assessment Module 109 may be configured to read the SNP results determined from a sample read of the microarray, and compare those results with a sample being sequenced on the sequencer 106. This allows a real time analysis of whether the sequencer is properly sequencing a predetermined sample. For example, if the microarray data indicates that a particular person has a SNP within a unique position in his or her genome, that data can be compared to sequence data coming from the sequencer 106. If that unique position is also being sequenced, then a comparison can be made to determine if the SNP in the sequenced sample matches with the SNP found in the same person's DNA when it was placed on the microarray. If the samples are accurate, then every unique SNP position from the microarray will match when that same unique section is sequenced on the sequencer 106. However, if a determination is made that the SNP sequences don't match, then the process can be flagged, paused, terminated or halted since the samples do not match one another, when they should.

The system 100 also includes a storage 110 which is configured to electronically store sequence information, Indexed equivalence classes, PHC 5mers, Array data and any other date involved in this process may be stored in the Storage 110. The system 100 also includes a display 111 for displaying results to a user.

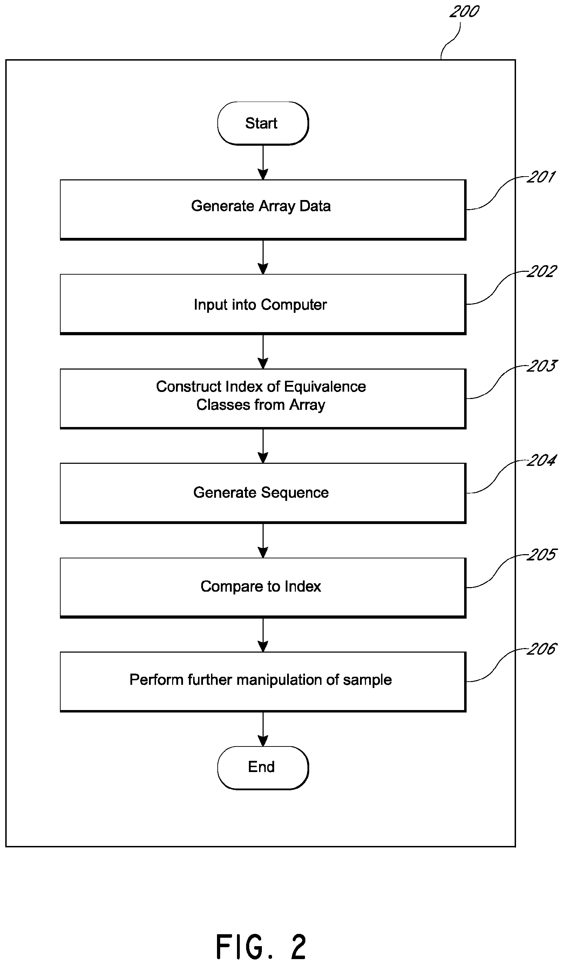

Figure 2 depicts a flow diagram of a process 200 of how to compare indexes of two samples through steps of a method consistent with some embodiments disclosed herein. At a state 201, array data is generated, which are indicative of the identity of one or more nucleotides in, for example, a 20mer from a particular biological sample which maps to a unique region of a genome and spans a single SNP, in a nucleic acid sample of interest. This array data is then inputted or read by a computer at a state 202. After the data is read by the computer, an index of equivalence classes is created at a state 203, informed by the sequence information from the array. The same biological sample can then be run on a sequencer to generate sequence data at a state 204. After, or at the same time that the sequencer is determining DNA sequences from the biological sample of interest, the process 200 moves to a state 205 wherein the sequence is compared to the Index generated at state 203 to determine whether the data from the array is consistent with the data from the sequencer. Depending on the results of this comparison carried out at state 205, one may then perform further manipulation to the sample at a state 206, such as generating more sequence data, performing post-sequencing analysis of the sequence, or discarding the sample and the sequence if the sequenced DNA does not seem to be matching the expected DNA sequences.

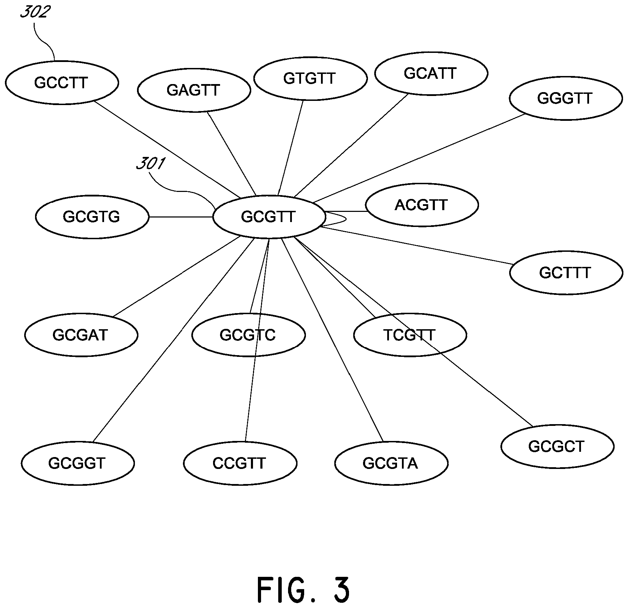

Figure 3 depicts an Equivalence Class built around a PHC 5mer 301, having a nucleotide sequence GCGTT. All members of the class, aside from oligo 301, differ from oligo 301 by no more than a single base. That is, each has a Hamming distance of 1 from the PHC oligo 301. Oligo 302, having a nucleotide sequence of GCCTT, for example differs from the PHC oligo 301 by a single G/C change at position 3 of the 5mer. PHC 5mer 301 differs by all other PHC 5mers by at least a Hamming code distance of 3, while other members of the equivalence class may differ from members of other equivalence classes by a Hamming code distance of 1.

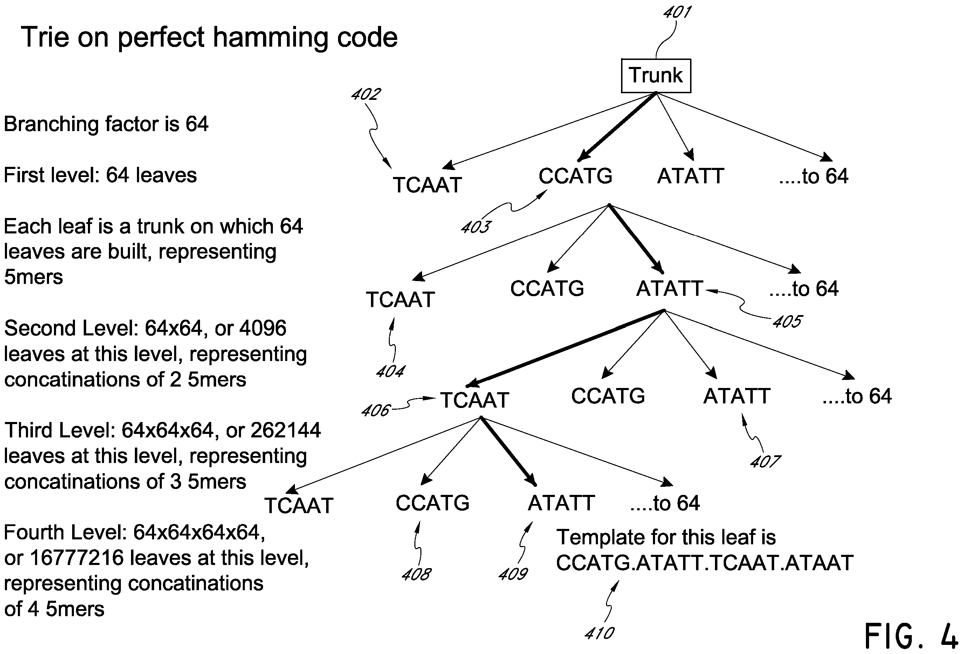

Figure 4 is a partial depiction of a four-level PHC trie used to rapidly analyze 20mer polynucleotide sequences. As is known to those of ordinary skill in the art, a "trie" is an ordered tree data structure that is used to store a dynamic set, or associative array, of data strings, such as nucleotides. Unlike a binary search tree, no node in the trie stores the key associated with that node. Instead, the data position in the tree defines the key with which it is associated. All the descendants of a node have a common prefix of the string associated with that node, and the root is associated with the empty string. Values are normally not associated with every node, only with leaves and some inner nodes that correspond to keys of interest. The trie starts from a Trunk 401, from which all 64 PHC 5mers directly branch to form the first-level leaves (402, 403, for example) of the trie. Only three of the 64 PHC leaves at this level are depicted.

In this depiction, first level leaf node 403 is shown as the trunk of a second level branching of 64 PHC 5mers (404 and 405 are examples). Only three of the 64 PHC leaves at this level branching from 403 are depicted, and none of the 63 other branching sets built off of the 63 other first level leaves are depicted.

Second level leaf 405 is shown as the trunk of a third level branching of 64 PHC 5mers (406 and 407 are examples). Only three of the 64 PHC leaves at this level branching from 405 are depicted, and none of the other branching sets built off of the (64² -1) other second level leaves are depicted.

Third level leaf 406 is shown as the trunk of a fourth level branching of 64 PHC 5mers (408 and 409 are examples). Only three of the 64 PHC leaves at this level branching from 406 are depicted, and none of the other branching sets built off of the (64³ -1) other second level leaves are depicted.

Also depicted is the path through 403, 405, 406 and 409, representing 20mer oligonucleotide 410.

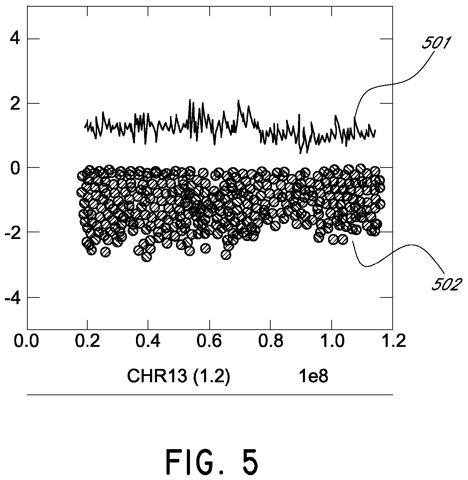

Figure 5 shows the implementation of an embodiment of the present disclosure. The copy number of a sequence of interest, chromosome 13, for example is determined through the methods disclosed herein. The ratio of ch13 copy number to a region elsewhere in the genome is depicted as 501. The relative number of 20mer oligonucleotide needles hitting the genomic sample analyzed is depicted in 502. This analysis is performed more rapidly and more quantitatively than present methods.

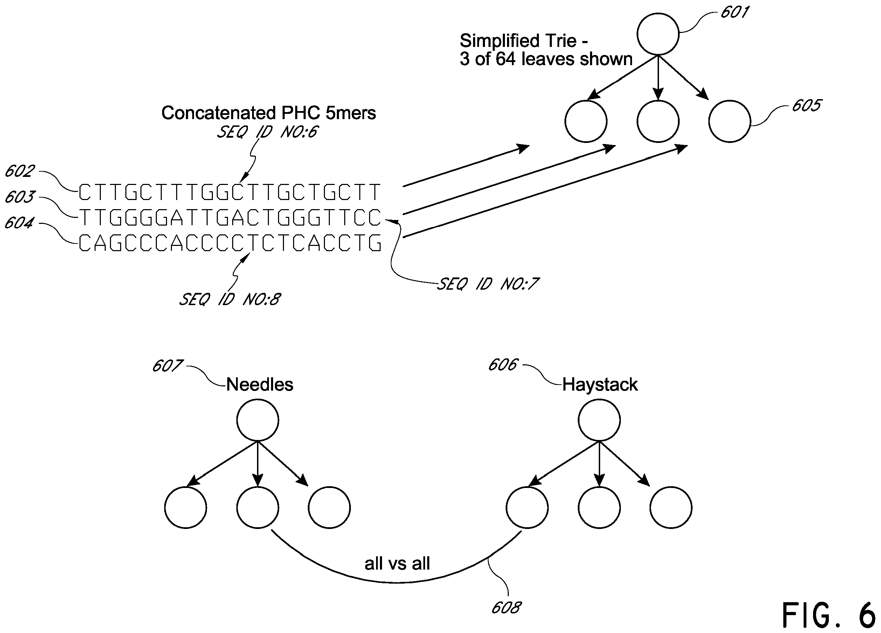

Figure 6 shows a stylized cartoon of an indexed 20mer data set. Endpoints corresponding to 20mer oligonucleotides 602, CTTGCTTTGGCTTGCTGCTT (SEQ ID NO:6); 603 TTGGGGATTGACTGGGTTCC (SEQ ID NO:7); and 604, CAGCCCACCCCTCTCACCTG (SEQ ID NO:8) are shown. The actual trie, it may be noted, has over 16 million leaves at its lowest level, 605. Figure 6 also shows how in some embodiments of the present disclosure, trie indices of large nucleic acid datasets 606 such as genomic sequence data may be compared with large 20mer needle data sets 607 in an all versus all comparison 608. This comparison is performed in some embodiments at over times much reduced compared to current methods.

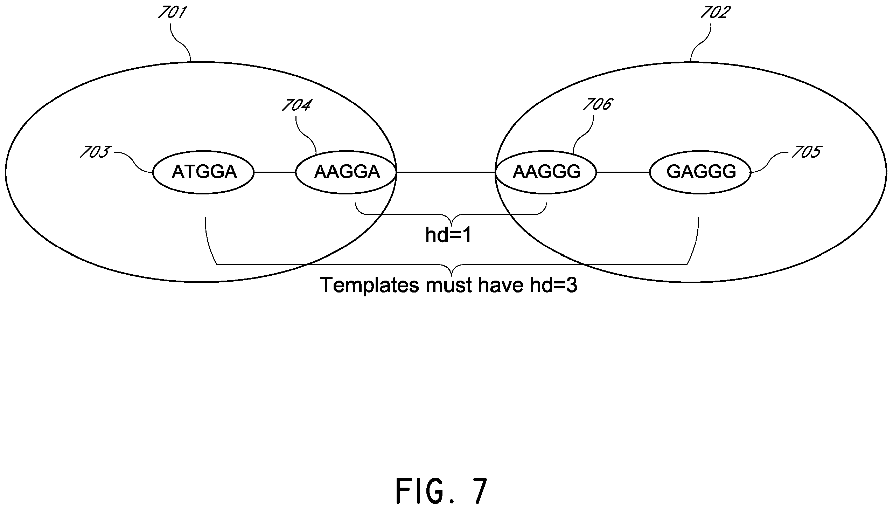

Figure 7 shows Hamming code distances between members of stylized index classes 701 and 702. PHC 5mer 703 ('ATGGA') is the core of equivalence class 701, which has 15 other 5mer members differing from 703 by a Hamming code distance of 1. Only one of these members, 704, is shown. Also shown is PHC 5mer 705 ('GAGGG'), the core of equivalence class 702. 702 has 15 other 5mer members differing from 705 by a Hamming code distance of 1. Only one of these members, 706, is shown. 703 and 705 have a Hamming code distance of 3, while 704 and 706 have a Hamming Code distance of 1. This figure illustrates that for non-PHC members of an equivalence class, some members of other equivalence classes may differ by a Hamming Code distance of 1. This necessitates the need to search the 30/64 equivalence classes that may differ include members differing by one from a sequence in an equivalence class.

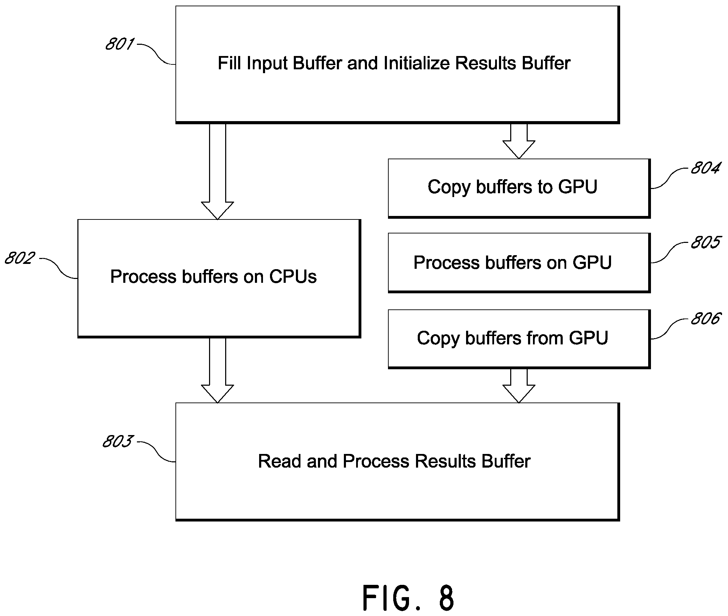

Figure 8 shows one embodiment of a process for filling input buffers and processing results in a sequence comparison system. The process begins by filling an input buffer and initializing a results buffer at a state 801. In one embodiment the buffers are processed on CPUs at state 802, and are read and their results processed at a state 803. In an alternate embodiment, the buffers are copied to a GPU at a state 804, processed on GPU at a state 805 and copied from a GPU at a state 806, after which they are read and their results processed at the state 803.

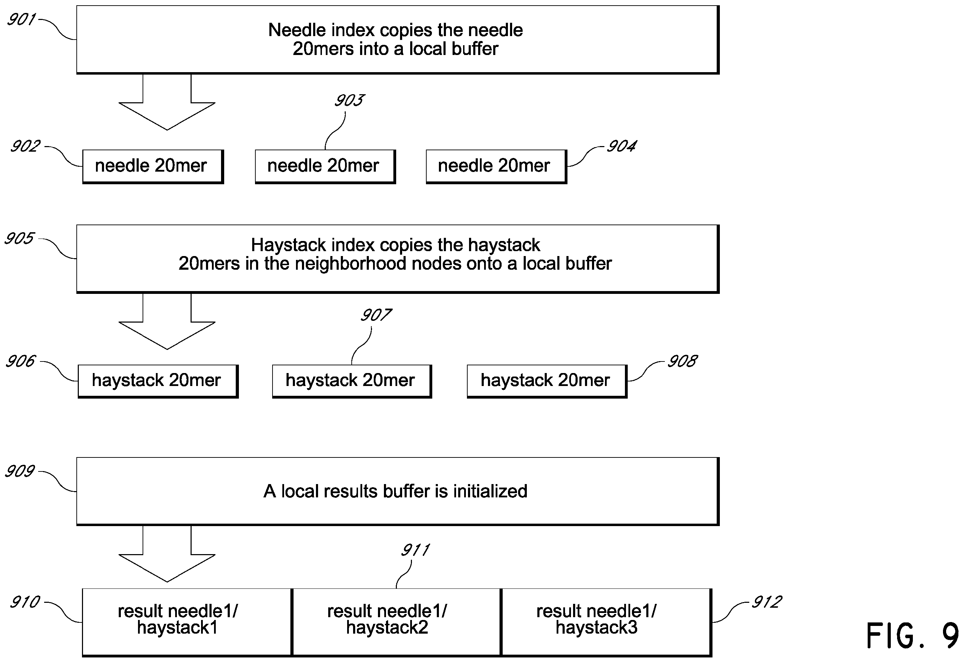

Figure 9 shows a block diagram of an embodiment of a process for determining results of a search for polynucleotide "needles" within a target "haystack" database of sequences. A needle index copies the needle 20mers into a local buffer at a state 901, storing needle 20mers at states 902-904. Similarly, a haystack index copies the haystack 20mers into a local buffer at a state 905, and storing needle 20mers at states 906-908. A local results buffer is initialized at a state 909, onto which result comparisons of, for example, needle 1 to haystack 1 (state 910), needle 1 to haystack 2 (state 911) and needle 1 to haystack 3 (state 912) are entered.

### EXAMPLES

### Example 1 Perfect Hamming Code template sequences and Equivalence class members

Perfect Hamming code sequences gtaag, cgaac, and tgata are given in Table 1. Each perfect Hamming code 5mer defines an equivalence class that includes 15 additional 5mers that differ from the PHC 5mer by one and only one nucleotide. The single base variation with the PHC 5mer is highlighted for illustrative purposes.

**TABLE 1.**

| PHC template sequence | Equivalence class of 5-mers covered by the template (base differing from the PHC 5mer is bold) |
|---|---|
| gtaag | gtaag ataag ; ttaag ; ctaag gaaag ; ggaag ; gcaag gttag ; gtgag ; gtcag gtatg ; gtagg ; gtacg gtaaa ; gtaat ; gtaac |
| cgaac | cgaac agaac tgaac ggaac caaac ccaac ctaac cgtac cggac cgcac cgatc cgagc cgacc cgaaa cgaat cgaag |
| tgata | tgata agata ; ggata ; cgata taata ; ttata ; tcata tgtta ; tggta ; tgcta tgaaa ; tgaga ; tgaca tgatt ; tgatg ; tgatc |

### Example 2 Concatenated PHC 5mers and equivalence class members

The PHC 5mers gtaag, cgaac and tgata were concatenated to from the 15mer oligonucleotide gtaagcgaactgata. Table 2 shows three example 15mers which are included in the equivalence class of this concatenated PHC 15mer.

**TABLE 2.**

| | | | |
|---|---|---|---|
| **15mer of PHC 5mers (SEQ ID NO:2)** | gtaag | cgaac | tgata |
| 15mers in equivalence class (read left to right) SEQ ID NOs:3, 4, and 5) | gtaac ataag ggaag | cgacc tgaac cgagc | tgatc tgaga agata |

## Claims

1. A computer implemented method of aligning a first 20mer oligonucleotide sequence to all 20mer fragments of a nucleotide data set comprising:
indexing all 5mer fragments of oligonucleotides into a first index of perfect Hamming code 5mers, wherein each said perfect Hamming code 5mer has a set of 15 additional 5mers which form an equivalence class with said perfect Hamming code 5mer such that each additional 5mer of said equivalence class differs from said perfect Hamming code 5mer by a Hamming code distance of 1, and wherein each said perfect Hamming code 5mer differs from all other perfect Hamming code 5mers by at least a Hamming code distance of 3;
configuring said first index into a first level of a four level trie having a branching factor of 64 such that each leaf of said first level represents a different perfect Hamming code 5mer of said first index and the equivalence class said perfect Hamming code 5mer defines;
configuring said trie such that each leaf of said first level is a trunk of a second index on a second level of said trie, said second index having the same structure as said first index, each leaf of said second level is a trunk of a third index on a third level of said trie, said third index having the same structure as said first index, and each leaf of said third level is a trunk of a fourth index on a fourth level of said trie, said fourth index having the same structure as said first index, such that said trie has a depth of 4 levels and 64⁴ leaves at said fourth level, and such that each 20mer fragment of said nucleotide data set maps to only one path from a 4th level leaf to the first level trunk of said trie, and wherein each unique trie path corresponds to a concatenation of four consecutive perfect Hamming code 5mers to form a 20mer fragment of said trie path;
assigning each 20mer fragment of said nucleotide data set to a unique trie path;
identifying a trie path corresponding to said first 20mer oligonucleotide sequence; and
comparing said 20mer fragments of said trie path to said first 20mer oligonucleotide sequence, wherein said comparing comprises:
comparing positions 1-5 of said first 20mer oligonucleotide sequence to the thirty equivalence classes at said first level each having at least one member which differs from at least one member of the equivalence class with positions 1-5 of said first 20mer oligonucleotide sequence by a Hamming distance of 1,
comparing positions 6-10 of said first 20mer oligonucleotide sequence to the thirty equivalence classes at said second level each having at least one member which differs from at least one member of the equivalence class with positions 6-10 of said first 20mer oligonucleotide sequence by a Hamming distance of 1,
comparing positions 11-15 of said first 20mer oligonucleotide sequence to the thirty equivalence classes at said third level each having at least one member which differs from at least one member of the equivalence class with positions 11-15 of said first 20mer oligonucleotide sequence by a Hamming distance of 1, and
comparing positions 16-20 of said first 20mer oligonucleotide sequence to the thirty equivalence classes at said fourth level each having at least one member which differs from at least one member of the equivalence class with positions 16-20 of said first 20mer oligonucleotide sequence by a Hamming distance of 1.

2. The computer implemented method of claim 1, wherein said first 20mer oligonucleotide maps to a unique region of a genomic reference sequence and wherein said first 20mer oligonucleotide sequence spans a single nucleotide polymorphic site.

3. The computer implemented method of claim 2, further comprising populating said nucleotide data set with sequence information from a nucleic acid sample sequence.

4. The computer implemented method of claim 3, wherein said first 20mer oligonucleotide sequence is derived from a sample, the method further comprising discarding said sample if said first 20mer oligonucleotide sequence is not identically present in said nucleic acid sample sequence.

5. The computer implemented method of claim 3, wherein said nucleic acid sample sequence comprises sequence from an incompletely sequenced nucleotide and said nucleotide sample is being sequenced concurrently with execution of said method.

6. The computer implemented method of claim 2, wherein said nucleotide data set and said first 20mer oligonucleotide sequence are derived from different sources.

7. The computer implemented method of claim 2, wherein said first 20mer oligonucleotide sequence is determined using a microarray hybridization or a sequencing method.

8. The computer implemented method of claim 1, wherein said first 20mer oligonucleotide sequence maps to a region that undergoes copy number variation in a population, and wherein hits to the sequence identical to said first 20mer oligonucleotide sequence are indicative of a copy number of said region in said sample.

9. A computer based system for aligning a first 20mer oligonucleotide sequence to all 20mer fragments of a nucleotide dataset comprising:
an index component adapted to index all 5mer fragments of oligonucleotides into a first index of perfect Hamming code 5mers, wherein each said perfect Hamming code 5mer has a set of 15 additional 5mers which form an equivalence class with said perfect Hamming code 5mer such that each additional 5mer of said equivalence class differs from said perfect Hamming code 5mer by a Hamming code distance of 1, and wherein each said perfect Hamming code 5mer differs from all other perfect Hamming code 5mers by at least a Hamming code distance of 3;
an iterative trie component-adapted to configure said first index into a first level of a four level trie having a branching factor of 64 such that each leaf of said first level represents a different perfect Hamming code 5mer of said index and the equivalence class it said perfect Hamming code 5mer defines, and such that each leaf of said first level is the trunk of a second index on a second level of said trie, said second index having the same structure as said first index, each leaf of said second level is the trunk of a third index on a third level of said trie, said third index having the same structure as said first index, and each leaf of said third level is the trunk of a fourth index on a fourth level of said trie, said fourth index having the same structure as said first index, such that said trie has a depth of 4 levels and 64⁴ leaves at said fourth level, and such that each 20mer fragment of said nucleotide dataset maps to only one path from a 4th level leaf to the first level trunk of said trie, wherein each unique trie path corresponds to a concatenation of four consecutive perfect Hamming code 5mers to form a 20mer fragment of said trie path;
an assignment component adapted to assign all 20mer fragments of said nucleotide dataset to a unique trie path; and
an identification component adapted to identify said trie path corresponding to said first 20mer oligonucleotide sequence;
an aligning component adapted to compare said 20mer fragments of said trie path to said first 20mer oligonucleotide sequence by:
comparing a first five positions of said first 20mer oligonucleotide sequence to the thirty equivalence classes at the first level each having at least one member which differs from at least one member of the equivalence class with a first five positions of said first 20mer oligonucleotide sequence by a Hamming distance of 1;
comparing a second five positions of said first 20mer oligonucleotide sequence to the thirty equivalence classes at the second level each having at least one member which differs from at least one member of the equivalence class with a second five positions of said first 20mer oligonucleotide by a Hamming distance of 1;
comparing a third five positions of said first 20mer oligonucleotide sequence to the thirty equivalence classes at the third level each having at least one member which differs from at least one member of the equivalence class with a third five positions of said first 20mer oligonucleotide sequence by a Hamming distance of 1; and
comparing a fourth five positions of said first 20mer oligonucleotide sequence to the thirty equivalence classes at the fourth level each having at least one member which differs from at least one member of the equivalence class with a fourth five positions of said first 20mer oligonucleotide sequence by a Hamming distance of 1; and
a report module adapted to report the results of said comparisons to a user.

10. The system of claim 9, wherein said nucleotide dataset comprises a sequence of a unique region of a genomic reference sequence and wherein said first 20mer oligonucleotide sequence maps to the sequence of the unique region and spans a single nucleotide polymorphic site.

11. The system of claim 9, wherein said nucleotide dataset comprises sequences from an incompletely sequenced nucleotide sample.

12. The system of claim 9, wherein said nucleotide dataset and said 20mer oligonucleotide sequence are derived from different sources.

13. The system of claim 11, further comprising a control component adapted to control a nucleic acid sequence generating apparatus in contact with said sample.

14. The system of claim 9, wherein said system has a component adapted to receive one or more sequences of the nucleotide dataset from a sequencing reaction or an oligonucleotide array hybridization reaction.

15. The system of claim 9, wherein said report indicates whether said first 20 oligonucleotide sequence is from a sample that is mislabeled.

## Patentansprüche

1. Computerimplementiertes Verfahren zum Abgleichen einer ersten 20-mer-Oligonucleotidsequenz mit allen 20-mer-Fragmenten eines Nucleotid-Datensatzes, das Folgendes umfasst:
das Indexieren aller 5-mer-Fragmente von Oligonucleotiden in einen ersten Index perfekter Hamming-Code-5-mere, wobei jedes der perfekten Hamming-Code-5-mere einen Satz aus 15 zusätzlichen 5-meren aufweist, die eine Äquivalenzklasse mit dem perfekten Hamming-Code-5-mer bilden, so dass sich jedes zusätzliche 5-mer der Äquivalenzklasse von dem perfekten Hamming-Code-5-mer durch einen Hamming-Code-Abstand von 1 unterscheidet, und wobei sich jedes perfekte Hamming-Code-5-mer von allen anderen perfekten Hamming-Code-5-meren durch zumindest einen Hamming-Code-Abstand von 3 unterscheidet;
das Konfigurieren des ersten Index in eine erste Ebene eines Tries mit vier Ebenen mit einem Verzweigungsfaktor von 64, so dass jedes Blatt der ersten Ebene für ein anderes perfektes Hamming-Code-5-mer des ersten Index steht und die Äquivalenzklasse das perfekte Hamming-Code-5-mer definiert;
das Konfigurieren des Tries, so dass jedes Blatt der ersten Ebene einen Stamm eines zweiten Index auf einer zweiten Ebene des Tries darstellt, wobei der zweite Index die gleiche Struktur wie der erste Index aufweist, jedes Blatt der zweiten Ebene einen Stamm eines dritten Index auf einer dritten Ebene des Tries darstellt, wobei der dritte Index die gleiche Struktur wie der erste Index aufweist, und jedes Blatt der dritten Ebene einen Stamm eines vierten Index auf einer vierten Ebene des Tries darstellt, wobei der vierte Index die gleiche Struktur wie der erste Index aufweist, so dass der Trie in der vierten Ebene eine Tiefe von 4 Ebenen und 64⁴ Blättern aufweist, und so dass jedes 20-mer-Fragment des Nucleotid-Datensatzes nur einen Pfad von einem Blatt der vierten Ebene zu dem Stamm der ersten Ebene des Tries abbildet, und wobei jeder eindeutige Triepfad einer Konkatenation von vier aufeinanderfolgenden perfekten Hamming-Code-5-meren entspricht, um ein 20-mer-Fragment des Triepfades zu bilden;
das Zuordnen jedes 20-mer-Fragments des Nucleotid-Datensatzes zu einem eindeutigen Triepfad;
das Identifizieren eines Triepfads, der der ersten 20-mer-Olgonucleotidsequenz entspricht; und
das Vergleichen der 20-mer-Fragmente des Tripfads mit der ersten 20-mer-Oligonucleotidsequenz, wobei das Vergleichen Folgendes umfasst:
das Vergleichen der Positionen 1 bis 5 der ersten 20-mer-Oligonucleotidsequenz mit den dreißig Äquivalenzklassen in der ersten Ebene, die jeweils zumindest ein Element aufweisen, das sich von zumindest einem Element der Äquivalenzklasse mit den Positionen 1 bis 5 der ersten 20-mer-Oligonucleotidsequenz um einen Hamming-Abstand von 1 unterscheidet,
das Vergleichen der Positionen 6 bis 10 der ersten 20-mer-Oligonucleotidsequenz mit den dreißig Äquivalenzklassen in der zweiten Ebene, die jeweils zumindest ein Element aufweisen, das sich von zumindest einem Element der Äquivalenzklasse mit den Positionen 6 bis 10 der ersten 20-mer-Oligonucleotidsequenz um einen Hamming-Abstand von 1 unterscheidet,
das Vergleichen der Positionen 11 bis 15 der ersten 20-mer-Oligonucleotidsequenz mit den dreißig Äquivalenzklassen in der dritten Ebene, die jeweils zumindest ein Element aufweisen, das sich von zumindest einem Element der Äquivalenzklasse mit den Positionen 11 bis 15 der ersten 20-mer-Oligonucleotidsequenz um einen Hamming-Abstand von 1 unterscheidet, und
das Vergleichen der Positionen 16 bis 20 der ersten 20-mer-Oligonucleotidsequenz mit den dreißig Äquivalenzklassen in der vierten Ebene, die jeweils zumindest ein Element aufweisen, das sich von zumindest einem Element der Äquivalenzklasse mit den Positionen 16 bis 20 der ersten 20-mer-Oligonucleotidsequenz um einen Hamming-Abstand von 1 unterscheidet.

2. Computerimplementiertes Verfahren nach Anspruch 1, wobei das erste 20-mer-Oligonucleotid eine eindeutige Region einer Genomreferenzsequenz abbildet und wobei die erste 20-mer-Oligonucleotidsequenz eine Einzelnucleotid-Polymorphismus-Stelle überspannt.

3. Computerimplementiertes Verfahren nach Anspruch 2, das weiters das Bestücken des Nucleotid-Datensatzes mit Sequenzinformationen von einer Nucleinsäureprobensequenz umfasst.

4. Computerimplementiertes Verfahren nach Anspruch 3, wobei die erste 20-mer-Oligonucleotidsequenz aus einer Probe stammt, wobei das Verfahren weiters das Verwerfen der Probe umfasst, wenn die erste 20-mer-Oligonucleotidsequenz in der Nucleinsäureprobensequenz nicht identisch vorhanden ist.

5. Computerimplementiertes Verfahren nach Anspruch 3, wobei die Nucleinsäureprobensequenz eine Sequenz eines unvollständig sequenzierten Nucleotids umfasst und die Nucleotidprobe gleichzeitig mit der Ausführung des Verfahrens sequenziert wird.

6. Computerimplementiertes Verfahren nach Anspruch 2, wobei der Nucleotid-Datensatz und die erste 20-mer-Oligonucleotidsequenz aus unterschiedlichen Quellen stammen.

7. Computerimplementiertes Verfahren nach Anspruch 2, wobei die erste 20-mer-Oligonucleotidsequenz unter Verwendung einer Mikroarray-Hybridisierung oder eines Sequenzierungsverfahrens bestimmt wird.

8. Computerimplementiertes Verfahren nach Anspruch 1, wobei die erste 20-mer-Oligonucleotidsequenz eine Region abbildet, die in einer Population eine Kopienanzahlvariation erfährt, und wobei Treffer in Bezug auf die Sequenz, die mit der ersten 20-mer-Oligonucleotidsequenz identisch ist, eine Kopienanzahl der Region in der Probe anzeigt.

9. Computerbasiertes System zum Abgleichen einer ersten 20-mer-Oligonucleotidsequenz mit allen 20-mer-Fragmenten eines Nucleotid-Datensatzes, das Folgendes umfasst:
eine Indexkomponente, die ausgelegt ist, um alle 5-mer-Fragmente von Oligonucleotiden in einen ersten Index perfekter Hamming-Code-5-mere zu indexieren, wobei jedes der perfekten Hamming-Code-5-mere einen Satz aus 15 zusätzlichen 5-meren aufweist, die eine Äquivalenzklasse mit dem perfekten Hamming-Code-5-mer bilden, so dass sich jedes zusätzliche 5-mer der Äquivalenzklasse von dem perfekten Hamming-Code-5-mer durch einen Hamming-Code-Abstand von 1 unterscheidet, und wobei sich jedes perfekte Hamming-Code-5-mer von allen anderen perfekten Hamming-Code-5-meren durch zumindest einen Hamming-Code-Abstand von 3 unterscheidet;
eine iterative Triekomponente, die ausgelegt ist, um den ersten Index in eine erste Ebene eines Tries mit vier Ebenen, der einen Verzweigungsfaktor von 64 aufweist, zu konfigurieren, so dass jedes Blatt der ersten Ebene für ein anderes perfektes Hamming-Code-5-mer des Index steht und die Äquivalenzklasse das perfekte Hamming-Code-5-mer definiert, und so dass jedes Blatt der ersten Ebene einen Stamm eines zweiten Index auf einer zweiten Ebene des Tries darstellt, wobei der zweite Index die gleiche Struktur wie der erste Index aufweist, jedes Blatt der zweiten Ebene einen Stamm eines dritten Index auf einer dritten Ebene des Tries darstellt, wobei der dritte Index die gleiche Struktur wie der erste Index aufweist, und jedes Blatt der dritten Ebene einen Stamm eines vierten Index auf einer vierten Ebene des Tries darstellt, wobei der vierte Index die gleiche Struktur wie der erste Index aufweist, so dass der Trie eine Tiefe von 4 Ebenen und 64⁴ Blättern in der vierten Ebene aufweist, und so dass jedes 20-mer-Fragment des Nucleotid-Datensatzes nur einen Pfad von einem Blatt vierter Ebene zu dem Stamm erster Ebene des Tries abbildet, und wobei jeder eindeutige Triepfad einer Konkatenation von vier aufeinander folgenden perfekten Hamming-Code-5-meren entspricht, um ein 20-mer-Fragment des Triepfades zu bilden;
eine Zuordnungskomponente, die ausgelegt ist, um alle 20-mer-Fragmente des Nucleotid-Datensatzes einem eindeutigen Triepfad zuzuordnen; und
eine Identifikationskomponente, die ausgelegt ist, um den Triepfad, der der ersten 20-mer-Olgonucleotidsequenz entspricht, zu identifizieren;
eine Abgleichkomponente, die ausgelegt ist, um die 20-mer-Fragmente des Triepfads mit der ersten 20-mer-Oligonucleotidsequenz durch Folgendes zu vergleichen:
das Vergleichen der ersten fünf Positionen der ersten 20-mer-Oligonucleotidsequenz mit den dreißig Äquivalenzklassen in der ersten Ebene, die jeweils zumindest ein Element aufweisen, das sich von zumindest einem Element der Äquivalenzklasse mit den ersten fünf Positionen der ersten 20-mer-Oligonucleotidsequenz um einen Hamming-Abstand von 1 unterscheidet,
das Vergleichen der zweiten fünf Positionen der ersten 20-mer-Oligonucleotidsequenz mit den dreißig Äquivalenzklassen in der zweiten Ebene, die jeweils zumindest ein Element aufweisen, das sich von zumindest einem Element der Äquivalenzklasse mit den zweiten fünf Positionen der ersten 20-mer-Oligonucleotidsequenz um einen Hamming-Abstand von 1 unterscheidet,
das Vergleichen der dritten fünf Positionen der ersten 20-mer-Oligonucleotidsequenz mit den dreißig Äquivalenzklassen in der dritten Ebene, die jeweils zumindest ein Element aufweisen, das sich von zumindest einem Element der Äquivalenzklasse mit den dritten fünf Positionen der ersten 20-mer-Oligonucleotidsequenz um einen Hamming-Abstand von 1 unterscheidet, und
das Vergleichen der vierten fünf Positionen der ersten 20-mer-Oligonucleotidsequenz mit den dreißig Äquivalenzklassen in der vierten Ebene, die jeweils zumindest ein Element aufweisen, das sich von zumindest einem Element der Äquivalenzklasse mit den vierten fünf Positionen der ersten 20-mer-Oligonucleotidsequenz um einen Hamming-Abstand von 1 unterscheidet; und
ein Berichtmodul, das ausgelegt ist, um einem Benutzer die Ergebnisse der Vergleiche anzuzeigen.

10. System nach Anspruch 9, wobei der Nucleotid-Datensatz eine Sequenz einer eindeutigen Region einer Genomreferenzsequenz umfasst und wobei die erste 20-mer-Oligonucleotidsequenz die Sequenz der eindeutigen Region abbildet und eine Einzelnucleotid-Polymorphismus-Stelle überspannt.

11. System nach Anspruch 9, wobei der Nucleotid-Datensatz Sequenzen einer unvollständig sequenzierten Nucleotidprobe umfasst.

12. System nach Anspruch 9, wobei der Nucleotid-Datensatz und die 20-mer-Oligonucleotidsequenz aus unterschiedlichen Quellen stammen.

13. System nach Anspruch 11, das weiters eine Steuerkomponente umfasst, die ausgelegt ist, um eine Vorrichtung zur Nucleinsäuresequenzerzeugung, die mit der Probe in Kontakt ist, zu steuern.

14. System nach Anspruch 9, wobei das System eine Komponente aufweist, die ausgelegt ist, um eine oder mehrere Sequenzen des Nucleotid-Datensatzes aus einer Sequenzierungsreaktion oder einer Oligonucleotidarray-Hybridisierungsreaktion zu empfangen.

15. System nach Anspruch 9, wobei der Bericht anzeigt, ob die erste 20-mer-Oligonucleotidsequenz aus einer Probe stammt, die falsch markiert ist.

## Revendications

1. Procédé mis en œuvre par ordinateur d'alignement d'une séquence de premier oligonucléotide 20mère avec tous les fragments 20mères d'un ensemble de données de nucléotide comprenant :
l'indexation de tous les fragments 5mères d'oligonucléotides dans un premier index de 5mères à code de Hamming parfait, dans lequel chacun desdits 5mères à code de Hamming parfait a un ensemble de 15 5mères supplémentaires qui forment une classe d'équivalence avec ledit 5mère à code de Hamming parfait de sorte que chaque 5mère supplémentaire de ladite classe d'équivalence diffère dudit 5mère à code de Hamming parfait par une distance de code de Hamming de 1, et dans lequel chacun desdits 5mères à code de Hamming parfait diffère de tous les autres 5mères à code de Hamming parfait par une distance de code de Hamming d'au moins 3 ;
la configuration dudit premier index dans un premier niveau d'un arbre à quatre niveaux ayant un facteur de ramification de 64 de sorte que chaque feuille dudit premier niveau représente un 5mère à code de Hamming parfait dudit premier index différent et la classe d'équivalence que ledit 5mère à code de Hamming parfait définit ;
la configuration dudit arbre de sorte que chaque feuille dudit premier niveau est un tronc d'un deuxième index sur un deuxième niveau dudit arbre, ledit deuxième index ayant la même structure que ledit premier index, chaque feuille dudit deuxième niveau est un tronc d'un troisième index sur un troisième niveau dudit arbre, ledit troisième index ayant la même structure que ledit premier index, et chaque feuille dudit troisième niveau est un tronc d'un quatrième index sur un quatrième niveau dudit arbre, ledit quatrième index ayant la même structure que ledit premier index, de sorte que ledit arbre a une profondeur de 4 niveaux et 64⁴ feuilles audit quatrième niveau, et de sorte que chaque fragment 20mère dudit ensemble de données de nucléotide correspond à un seul trajet d'une feuille de 4^{e} niveau au tronc de premier niveau dudit arbre, et dans lequel chaque trajet d'arbre unique correspond à une concaténation de quatre 5mères à code de Hamming parfait consécutifs pour former un fragment 20mère dudit trajet d'arbre ;
l'assignation de chaque fragment 20mère dudit ensemble de données de nucléotide à un trajet d'arbre unique ;
l'identification d'un trajet d'arbre correspondant à ladite séquence de premier oligonucléotide 20mère ; et
la comparaison desdits fragments 20mères dudit trajet d'arbre à ladite séquence de premier oligonucléotide 20mère, dans lequel ladite comparaison comprend :
la comparaison des positions 1 à 5 de ladite séquence de premier oligonucléotide 20mère aux trente classes d'équivalence audit premier niveau ayant chacune au moins un membre qui diffère d'au moins un membre de la classe d'équivalence avec les positions 1 à 5 de ladite séquence de premier oligonucléotide 20mère par une distance de Hamming de 1,
la comparaison des positions 6 à 10 de ladite séquence de premier oligonucléotide 20mère aux trente classes d'équivalence audit deuxième niveau ayant chacune au moins un membre qui diffère d'au moins un membre de la classe d'équivalence avec les positions 6 à 10 de ladite séquence de premier oligonucléotide 20mère par une distance de Hamming de 1,
la comparaison des positions 11 à 15 de ladite séquence de premier oligonucléotide 20mère aux trente classes d'équivalence audit troisième niveau ayant chacune au moins un membre qui diffère d'au moins un membre de la classe d'équivalence avec les positions 11 à 15 de ladite séquence de premier oligonucléotide 20mère par une distance de Hamming de 1, et
la comparaison des positions 16 à 20 de ladite séquence de premier oligonucléotide 20mère aux trente classes d'équivalence audit quatrième niveau ayant chacune au moins un membre qui diffère d'au moins un membre de la classe d'équivalence avec les positions 16 à 20 de ladite séquence de premier oligonucléotide 20mère par une distance de Hamming de 1.

2. Procédé mis en œuvre par ordinateur selon la revendication 1, dans lequel ledit premier oligonucléotide 20mère correspond à une région unique d'une séquence de référence génomique et dans lequel ladite séquence de premier oligonucléotide 20mère couvre un site de polymorphisme d'un seul nucléotide.

3. Procédé mis en œuvre par ordinateur selon la revendication 2, comprenant en outre le peuplement dudit ensemble de données de nucléotide avec des informations de séquence d'une séquence d'échantillon d'acide nucléique.

4. Procédé mis en œuvre par ordinateur selon la revendication 3, dans lequel ladite séquence de premier oligonucléotide 20mère est dérivée d'un échantillon, le procédé comprenant en outre le rejet dudit échantillon si ladite séquence de premier oligonucléotide 20mère n'est pas présente de façon identique dans ladite séquence d'échantillon d'acide nucléique.

5. Procédé mis en œuvre par ordinateur selon la revendication 3, dans lequel ladite séquence d'échantillon d'acide nucléique comprend une séquence d'un nucléotide incomplètement séquencé et ledit échantillon de nucléotide est séquencé concomitamment avec l'exécution dudit procédé.

6. Procédé mis en œuvre par ordinateur selon la revendication 2, dans lequel ledit ensemble de données de nucléotide et ladite séquence de premier oligonucléotide 20mère sont dérivés de sources différentes.

7. Procédé mis en œuvre par ordinateur selon la revendication 2, dans lequel ladite séquence de premier oligonucléotide 20mère est déterminée au moyen d'un procédé d'hybridation sur microréseau ou de séquençage.

8. Procédé mis en œuvre par ordinateur selon la revendication 1, dans lequel ladite séquence de premier oligonucléotide 20mère correspond à une région qui subit une variation du nombre de copies dans une population, et dans lequel des correspondances avec la séquence identique à ladite séquence de premier oligonucléotide 20mère sont indicatrices d'un nombre de copies de ladite région dans ledit échantillon.

9. Système informatique d'alignement d'une séquence de premier oligonucléotide 20mère avec tous les fragments 20mères d'un ensemble de données de nucléotide comprenant :
un composant d'indexation adapté pour indexer tous les fragments 5mères d'oligonucléotides dans un premier index de 5mères à code de Hamming parfait, dans lequel chacun desdits 5mères à code de Hamming parfait a un ensemble de 15 5mères supplémentaires qui forment une classe d'équivalence avec ledit 5mère à code de Hamming parfait de sorte que chaque 5mère supplémentaire de ladite classe d'équivalence diffère dudit 5mère à code de Hamming parfait par une distance de code de Hamming de 1, et dans lequel chacun desdits 5mères à code de Hamming parfait diffère de tous les autres 5mères à code de Hamming parfait par une distance de code de Hamming d'au moins 3 ;
un composant d'arbre itératif adapté pour configurer ledit premier index dans un premier niveau d'un arbre à quatre niveaux ayant un facteur de ramification de 64 de sorte que chaque feuille dudit premier niveau représente un 5mère à code de Hamming parfait dudit premier index différent et la classe d'équivalence que ledit 5mère à code de Hamming parfait définit, et de sorte que chaque feuille dudit premier niveau est le tronc d'un deuxième index sur un deuxième niveau dudit arbre, ledit deuxième index ayant la même structure que ledit premier index, chaque feuille dudit deuxième niveau est le tronc d'un troisième index sur un troisième niveau dudit arbre, ledit troisième index ayant la même structure que ledit premier index, et chaque feuille dudit troisième niveau est le tronc d'un quatrième index sur un quatrième niveau dudit arbre, ledit quatrième index ayant la même structure que ledit premier index, de sorte que ledit arbre a une profondeur de 4 niveaux et 64⁴ feuilles audit quatrième niveau, et de sorte que chaque fragment 20mère dudit ensemble de données de nucléotide correspond à un seul trajet d'une feuille de 4^{e} niveau au tronc de premier niveau dudit arbre, dans lequel chaque trajet d'arbre unique correspond à une concaténation de quatre 5mères à code de Hamming parfait consécutifs pour former un fragment 20mère dudit trajet d'arbre ;
un composant d'assignation adapté pour assigner tous les fragments 20mères dudit ensemble de données de nucléotide à un trajet d'arbre unique ; et
un composant d'identification adapté pour identifier ledit trajet d'arbre correspondant à ladite séquence de premier oligonucléotide 20mère ;
un composant d'alignement adapté pour comparer lesdits fragments 20mères dudit trajet d'arbre à ladite séquence de premier oligonucléotide 20mère par :
comparaison de premières cinq positions de ladite séquence de premier oligonucléotide 20mère aux trente classes d'équivalence au premier niveau ayant chacune au moins un membre qui diffère d'au moins un membre de la classe d'équivalence avec des premières cinq positions de ladite séquence de premier oligonucléotide 20mère par une distance de Hamming de 1 ;
comparaison de deuxièmes cinq positions de ladite séquence de premier oligonucléotide 20mère aux trente classes d'équivalence au deuxième niveau ayant chacune au moins un membre qui diffère d'au moins un membre de la classe d'équivalence avec des deuxièmes cinq positions dudit premier oligonucléotide 20mère par une distance de Hamming de 1 ;
comparaison de troisièmes cinq positions de ladite séquence de premier oligonucléotide 20mère aux trente classes d'équivalence au troisième niveau ayant chacune au moins un membre qui diffère d'au moins un membre de la classe d'équivalence avec des troisièmes cinq positions de ladite séquence de premier oligonucléotide 20mère par une distance de Hamming de 1 ; et
comparaison de quatrièmes cinq positions de ladite séquence de premier oligonucléotide 20mère aux trente classes d'équivalence au quatrième niveau ayant chacune au moins un membre qui diffère d'au moins un membre de la classe d'équivalence avec des quatrièmes cinq positions de ladite séquence de premier oligonucléotide 20mère par une distance de Hamming de 1 ; et un module de rapport adapté pour rapporter les résultats desdites comparaisons à un utilisateur.

10. Système selon la revendication 9, dans lequel ledit ensemble de données de nucléotide comprend une séquence d'une région unique d'une séquence de référence génomique et dans lequel ladite séquence de premier oligonucléotide 20mère correspond à la séquence de la région unique et couvre un site de polymorphisme d'un seul nucléotide.

11. Système selon la revendication 9, dans lequel ledit ensemble de données de nucléotide comprend des séquences d'un échantillon de nucléotide incomplètement séquencé.

12. Système selon la revendication 9, dans lequel ledit ensemble de données de nucléotide et ladite séquence oligonucléotidique de 20mère sont dérivés de sources différentes.

13. Système selon la revendication 11, comprenant en outre un composant de contrôle adapté pour contrôler un appareil de génération de séquence d'acide nucléique en contact avec ledit échantillon.

14. Système selon la revendication 9, dans lequel ledit système comporte un composant adapté pour recevoir une ou plusieurs séquences de l'ensemble de données de nucléotide provenant d'une réaction de séquençage ou d'une réaction d'hybridation sur réseau d'oligonucléotides.

15. Système selon la revendication 9, dans lequel ledit rapport indique si ladite séquence de premier oligonucléotide 20mère provient d'un échantillon qui est incorrectement étiqueté.
